# EUROPEAN PATENT SPECIFICATION

(11) **EP 2 575 666 B1**
(45) Date of publication and mention of the grant of the patent: **13.08.2014**
(21) Application number: 11730052.5
(22) Date of filing: 31.05.2011
(51) Int. Cl.: A61B 19/02, A61B 1/00, A61L 2/28, B65B 31/04, B65D 81/20

(54) **METHODS OF PACKING MEDICAL DEVICES**
VERFAHREN ZUR VERPACKUNG VON MEDIZINPRODUKTEN
PROCÉDÉS D'EMBALLAGE DE DISPOSITIFS MÉDICAUX

(30) Priority: 02.06.2010 GB 201009230
(43) Date of publication of application: 10.04.2013
(73) Proprietor: GETINGE UK LIMITED, Waterbeach Cambridge Cambridgeshire CB25 9QP (GB)
(72) Inventor: BAKER, Simon, Cambridgeshire CB25 9QP (GB)
(74) Representative: Hartwell, Ian Peter
(86) International application number: PCT/GB2011/051024
(87) International publication number: WO 2011/151641

(56) References cited:
- EP-A2- 1 481 693
- WO-A1-2010/046617
- US-A- 6 161 695

## Description

### TECHNICAL FIELD

The present invention relates to methods of packaging medical devices, in particular but not exclusively to methods of packaging endoscopes, particularly flexible endoscopes.

### BACKGROUND ART

Flexible endoscopes are expensive, complex, reusable instruments that require unique consideration with respect to decontamination, storage and transportation between each patient procedure. In addition to the external surface of an endoscope, the internal 'channels' for air, water, aspiration, accessories etc are exposed to body fluids and other contaminants. Endoscopes are routinely exposed to mucus and other gastrointestinal secretions, blood, saliva, faeces, bile, and sometimes pus all of which can aid to the cross infection of microorganisms from one patient to the next.

In contrast to rigid endoscopes and most reusable accessories, flexible endoscopes are thermolabile (heat sensitive) and cannot be sterilized which is the standard procedure for most instruments, known as the 'terminal process'. Sterilisation is defined as the complete destruction of all micro-organisms including bacterial spores. Sterilisation is required for devices that are normally used in sterile areas of the body (e.g. laparoscopes, microsurgical instruments). Flexible endoscopes (which make contact with mucous membranes but do not ordinarily penetrate normally sterile areas of the body) are generally reprocessed by high level disinfection rather than sterilisation in order to kill bacteria, viruses, mycobacteria and some spores - see e.g. UK Medicines and Healthcare products Regulatory Agency Medical Device Alert 2004/028. Most flexible gastrointestinal endoscopes would not withstand the conditions normally used in a steam sterilisation process.

Medical apparatus such as endoscopes are typically exposed to two different types of bacteria. Aerobic bacteria grow in the presence of O₂, and are the most common causes of clinical infection. Aerobic bacteria such as Aspergillus (see DSC), Staphylococcus and Pseudomonas Aeruginosa cause blood infection and are harmful to human beings especially persons believed to have weak immune systems. An aerobic bacterium is an organism that comprises a metabolism based on oxygen. It is a type of bacteria that requires oxygen for its growth and survival. Aerobic bacteria use oxygen for oxidizing the substrates such as fats or sugars for obtaining energy.

Anaerobic bacteria are a type of bacteria that grow in places which are starved of oxygen. Such bacteria infect deep lacerations, deep tissues, and internal organs. Infections are marked by bad-smelling pus, the formation of abscesses, and the destruction of tissue. The bacteria are most often located in the mouth, gastrointestinal tract, vagina, and on the skin. Examples are Staphylococcus aureus and C. diff (Clostridium difficile). Certain anaerobic bacteria, known as obligate anaerobes, die in the presence of oxygen. Facultative anaerobes, on the other hand, can adapt to both aerobic and anaerobic habitats. This versatility is what gives the facultative anaerobe E. coli (Escherichia coli) its ability to adapt to its intestinal (anaerobic) and its extra-intestinal (aerobic or anaerobic) habitats.

The United Kingdom Department of Health has issued a number of HSC's (Health Service Circulars), guidelines and legislations to be adhered to for the treatment of flexible endoscopes between patient uses, much of which is documented in the BSG (British Society of Gastroenterology) Endoscopy decontamination guidelines. Between each patient use the following steps should be taken:
a. Decontamination should begin as soon as the endoscope has been removed from the patient.
b. Before the endoscope is detached from the light source/videoprocessor a preliminary cleaning routine should be undertaken.
a. The endoscope is then detached from the light source/videoprocessor, removed to the reprocessing room and attached to a leakage tester system (not exceeding 1 bar pressure as per the endoscope manufacturers recommendations) to check the integrity of all channels or outer casing for bite damage or any other damage before reprocessing.
b. The next stage is manual cleaning and rinsing of all exposed internal and external surfaces. A low-foaming detergent that has been specifically designated for medical instrument cleaning should be used.
c. The next stage is automated washing and high level disinfection with a liquid chemical germicide within an AER (Automated Endoscope Reprocessor) followed by rinsing with sterile grade water.

The scope may now be used again, or hung up ready for use within 3 hours or transported to another department and hung ready for use within 3 hours, alternatively it may be placed in a DSC (Drying and Storage Cabinet) for 3 to 7 days (which becomes the terminal process) ready for immediate use. Many units are now using purpose built DSC's, which have been shown to prevent colonisation of endoscope channels over time periods ranging from 72hours to 7 days. These DSCs are designed to deliver high efficiency particulate filtered air to the internal channels of the endoscope at the appropriate temperature and flow rate. According to the manufacturers, their use avoids the need for endoscopes to undergo early morning repeat decontamination cycles.

Specifically, the test method used on the DSC states that following storage the acceptable contamination of the internal channels of endoscopes shall be less than 10cfu (Colony Forming Units) and no pathogenic microorganisms, aspergillus (aerobic) or any other filamentous fungi shall be found. Within an AER 10 cfu/ml is the maximum allowable level within the European Standard BS EN ISO 15883 for final rinse water during the terminal process..

The use of such DSCs positioned around a hospital within different departments has allowed endoscope decontamination to become centralised within hospitals, which is deemed to be best practice. The down side to this new practice is that safe and aseptic transportation of endoscopes from one department to another is not readily available or very expensive and slow to prepare and added to this, the purchase of many DSC's is prohibitive. Moreover, guidelines require that when transporting endoscopes to and from areas outside the endoscopy unit, they must be transferred in a covered rigid receptacle, not only to avoid damage to the endoscope, but also to protect the cleanliness of the scope and to protect staff and the public when returning potentially contaminated scopes.

US6161695 discloses a method for protectively packaging sterilizable materials. A product to be packaged is placed within a semi-rigid, foldable packaging tray to form a package insert. The package insert is then placed within a heat-sealable container, such as a polymeric film envelope. Vacuum force is applied to evacuate the vacuum sealable container and to seal the packaging insert within the vacuum sealable container. The vacuum sealed container may then be placed within an outer, flexible packaging container and heat sealed therein. The entire package is generally sterilized after heat sealing within the outer, flexible package.

### DISCLOSURE OF THE INVENTION

According to the present invention, there is provided a method of packaging a medical device , comprising the steps of:
placing the medical device in a bag;
removing air from the bag by applying a vacuum of less than 1 bar; and
hermetically sealing the bag while the vacuum is being applied such that the concentration of oxygen in the hermetically sealed bag is 16% +/- 0.5% by volume.

Such a level of oxygen stops the growth of both obligate anaerobic bacteria and aerobic bacteria on the surface and within the inner channels of flexible endoscopes during transportation and/or storage, prolonging the aseptic storage and safe transportation of medical devices. The method is particularly appropriate for packaging non-sterile medical devices, especially endoscopes and in particular flexible endoscopes which, by their nature, cannot be sterilized but only subjected to high level disinfection.

The method may be for packaging a medical device having a sealed chamber, in which case the method may comprise the step of applying a vacuum to the interior of the bag such that the pressure difference between the sealed chamber and the interior of the bag does not exceed 1 bar.

Medical devices having chambers sealed from external conditions include flexible endoscopes where the internal workings, which may include a camera and a light, are sealed from the external environment of mucus, etc., thereby defining a sealed chamber. Subjecting the chamber seal to a pressure difference in excess of 1 bar can cause it to fail, potentially allowing the external environment to contaminate the internal workings or vice versa.

The vacuum in the bag at the time the bag is hermetically sealed is preferably about 250mbar below the ambient pressure (typically 1 bar (10⁵ Pa)), i.e. about 750mbar absolute. This is to be contrasted with vacuum packaging in the food industry where higher levels of vacuum in the range 700mbar to 1 bar below ambient are applied and obligate anaerobic bacteria are less prevalent.

The step of hermetically sealing the bag may comprise the creation of at least two seals in series, in particular three seals in series ("triple sealed").

The method may comprise the further step of subjecting the medical device to high level disinfection prior to placing the device in a bag. Prior to such high level disinfection, the method may comprise the step of placing a contaminated ("dirty") endoscope in a bag and sealing the bag, thereby removing any cross infection risks for both staff and patients when transporting dirty endoscopes from one area to another or from one hospital to another.

The method may comprise the further step of ensuring that the exposed surfaces of the medical device are dry before placing the medical device in a bag. This enables prolonged aseptic storage of the device. Drying of the device may be achieved by placing the medical device in a DSC prior to placing the medical device in the bag.

The method may comprise the further step of placing the medical device on a tray before placing the device and tray into the bag. The use of the tray helps to protect the scope from any abnormal contours under the effect of the vacuum. The method may comprise the step of providing a tray having a supporting surface with a recess formed therein and placing the device such that one part of the device is supported by the surface and another part of the device lies in the recess. The method may comprise the step of providing a bridge member on the tray to cover part of the device.

The method may comprise the further step of placing the medical device in a further, sterile bag before placing it in a bag to which the vacuum is applied. The use of a sterile bag inside the vacuum bag ensures the scope is not compromised.

The method may comprise the further step of enclosing the hermetically sealed, device-containing bag in a rigid case, thereby providing further protection of the device and its sealed bag. The additional use of a tray ensures the package shape fits perfectly into the transportation case every time.

Yet further protection may be provided by placing the rigid case in an enclosure configured to accommodate a plurality of such cases.

The method may comprise the further step of monitoring the vacuum in the bag at the time the bag is hermetically sealed, comparing the monitored vacuum with a predetermined value and generating a signal depending on the result of said comparison.

The monitoring of the vacuum may be carried out independently of the air removal step, which may require its own monitoring. Such an independent monitoring system (IMS) may be independent of the packaging device's microprocessor controller and may be fitted with its own set of probes used to monitor all cycle/process parameters that are critical to the process. The resulting cycle validation data/signal may be passed to a PC or mass storage device and information can then be saved to the patient's file, as often required by law.

Alternatively, the signal may cause a printer to generate one or more corresponding labels which may then be attached to the bag and/or the case.

### BRIEF DESCRIPTION OF THE DRAWINGS

An embodiment of the invention will now be described by way of example with reference to the accompanying drawings, in which:
Figure 1A illustrates a medical device when subject to the packaging process;
Figure 1B is a detailed view of the underside of the cover 60 in figure 1A;
Figures 2A and 2B are detail views of the tip and opposite ends of the insertion tube of a typical endoscope;
Figure 3A is a graph showing the reduction in microbial populations for a "dry" mock endoscope containing small amounts of C.difficile microbes and packaged in accordance with the invention;
Figure 3B is a graph showing the reduction in microbial populations for a "wet" mock endoscope containing small amounts of P.aeruginosa microbes and packaged in accordance with the invention;
Figure 3C is a graph showing the reduction in microbial populations for a "wet" mock endoscope containing small amounts of C.difficile microbes and packaged in accordance with the invention;
Figure 3D is a graph showing the reduction in microbial populations for a "wet" mock endoscope containing small amounts of S.aureus microbes and packaged in accordance with the invention;
Figure 4A shows an endoscope packaged according to the invention and placed in a rigid case;
Figure 4B is a plan view of an endoscope packaged in accordance with the invention;
Figure 4C is a perspective view of a detail of figure 4B;
Figure 5 shows several rigid cases in an enclosure.
Figure 6 is a schematic of a monitoring system.

### DETAILED DESCRIPTION OF SPECIFIC EMBODIMENTS

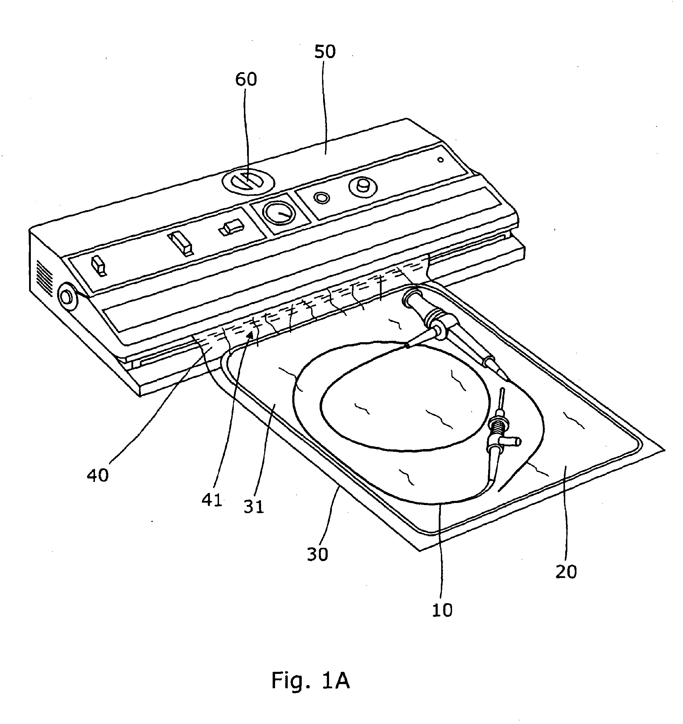

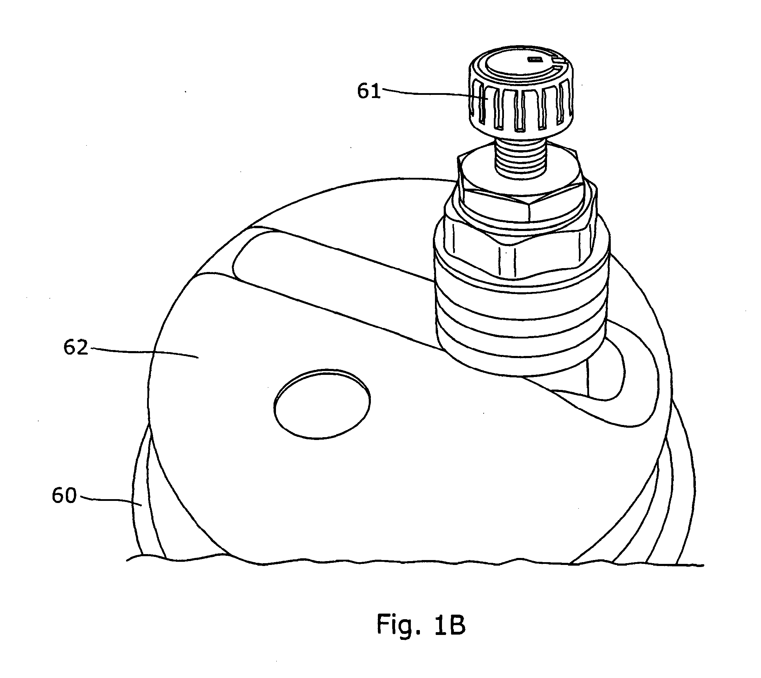

Figure 1 shows a medical device in the form of an endoscope 10 placed on a tray 20 and then in a bag 30. The mouth 40 of the bag is placed in a combined vacuum pump and sealing unit 50 which removes air and any moisture from the interior 31 of the bag before hermetically sealing the bag with a triple seal (indicated at 41) whilst the vacuum is being applied. The material of the bag is substantially impermeable to gas, having an oxygen permeability of about 50 cm³/m².d.bar or less, thereby ensuring that the vacuum in the bag is maintained. One suitable bag material is a polyamide/polyethylene laminate of the kind sold e.g. by Lava Vacuumverpackung under the name "EK-flex N90 embossed". This material has an oxygen permeability of 50 cm³/m².d.bar (per DIN 53380). It also has a water vapour transmission rate of <3.0 g/m².d (per DIN 53122).

The material of the bag typically lies in the range 80µm to 120µm, the particular material mentioned above having a total gauge of 90µm (per DIN53370) and a weight per area of 88 g/m² (per DIN ISO 536). Although the interior of the bag is initially clean by virtue of the bag manufacturing process (in particular the temperatures involved), the interior of the bag need not be sterile before use.

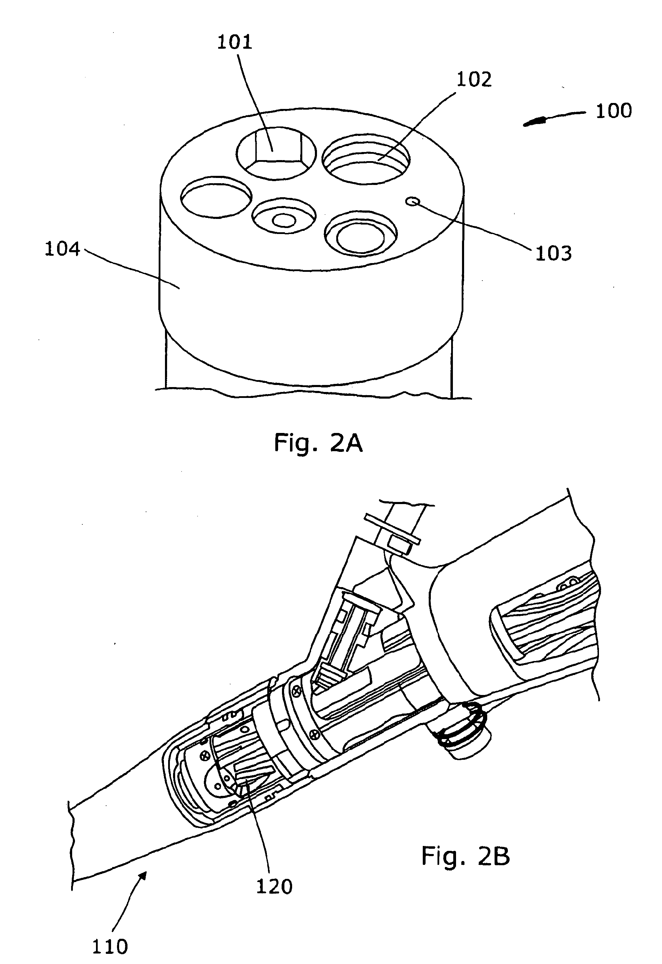

Figure 2A shows the highly flexible distal tip 100 of the insertion tube having an external surface 104 as well as channels or lumens for supply of air or water (101), for the purposes of biopsy or the application of suction (102) and for a water jet (103). Figure 2B shows the internal workings 110 at the other end of the insertion tube, in particular one or more chambers 120 which are sealed to prevent leakage into or out of the endoscope etc. during operation. Flexible endoscopes are subjected to a leak test between patient uses and the maximum pressure used for this purpose is less than 1 bar, higher pressures could easily damage the highly flexible distal tip of the insertion tube for instance.

The vacuum applied by the unit to the bag at the time the bag is hermetically sealed is about 250mbar below the ambient pressure of 1 bar (10⁵ Pa), i.e. about 750mbar absolute. The level of vacuum is determined by an adjustable valve 61 which, as illustrated in figure 1B, is located on the underside 62 of a removable cover 60 on the outside of the vacuum pump and sealing unit 50 so as to be readily accessible for maintenance and quality control purposes. Such a vacuum level avoids a pressure difference between internal chambers 120 and the inside of the bag 30 exceeding I bar. This level of vacuum in the bag also results in the sealed bag containing oxygen at a level sufficiently high to suppress the growth of obligate anerobic bacteria but sufficiently low to deteriorate aerobic bacteria. Using the same sized tray in the same sized bag at the same level of vacuum ensures reproducibility: typical tray dimensions are around 450mm x 350mm x 25mm, while typical bag dimensions are around 600mm length and 400mm to 550 mm width. Endoscopes typically range in length from around 3m to around 1.5m, the former corresponding to a gastrointestinal (GI) device having an insertion tube of up to 15mm diameter and around 1.7m length connected to a handle of around 0.2m length and attached thereto an umbilical or light source of around 1m length. The lower end of the range corresponds to an ear, nose and throat (ENT) device having an insertion tube of around 2mm diameter and 0.3 m length, a handle of 0.17m length and an umbilical of around 1m length.

Such a level of vacuum need not kill microorganisms within the system, just reduce their rate of proliferation, thereby maintaining the endoscope below the prescribed level of contamination (e.g. 10CFU/ml) for longer.

The process can be used with "wet" endoscopes that have undergone automated washing and high level disinfection but that have not been dried. It can also be used with endoscopes that have been processed through a validated AER and dried within an Endoscope Drying Cabinet, i.e. "dry" endoscopes.

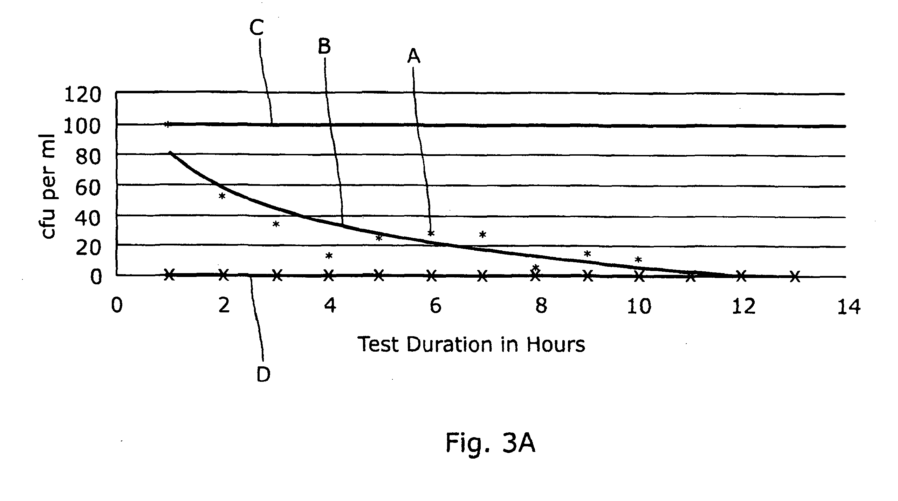

Figure 3A illustrates the reduction in microbial populations (measured in colony forming units / millilitre) for surrogate/mock "dry" endoscopes containing small amounts of microbes packed in the manner set out above and evaluated at regular intervals over a 30 day period. Pieces of 2mm ID PTFE were cut into 1.5m and 2m lengths (to create Surrogate Device test pieces) and sealed in plastic packs and irradiated for sterility. A culture was prepared with a known concentration of C.difficile at approximately 10³ suspended in Nutrient Broth. 0.03% Bovine Serum albumin was added to the culture. A Surrogate Device from a sterilized pack was aseptically removed and inoculated with the aforementioned suspension. The surrogate devices were then placed for 2 hours in a 22 incubator on a revolving platform to dry the suspension. Microorganisms were tested separately. Surrogate Devices were packed in a controlled ambient environment at 20°C ± 2°C, in a "dirty" laboratory with 8 air room changes per hour. A positive control for each organism was tested in a Surrogate Device for both 1.5m and 2m lengths to establish the amount of bacteria contained in each device after the incubation drying period. The inoculated test tubes [Surrogate Device] were then packed in the manner set out above together with one un-inoculated tube (negative control) for each set of shelf life tests.

To serve as a comparison between devices packed according to the invention and uncontrolled devices, a positive control set for each test day was inoculated. Devices were placed in an unsealed bag and left in ambient air conditions [20°C ± 2°C, in a "dirty" laboratory with 8 air room changes per hour].

Those bags prepared in accordance with the invention were pressure tested for seal integrity using a pressure/vacuum testing system. After the set time intervals listed above, the seal integrity was tested before opening again using a pressure/ vacuum testing system. The test Surrogate Devices, the inoculated unpacked surrogate devices and the packed negative control tubes were then examined after designated storage period for the presence of viable organisms.

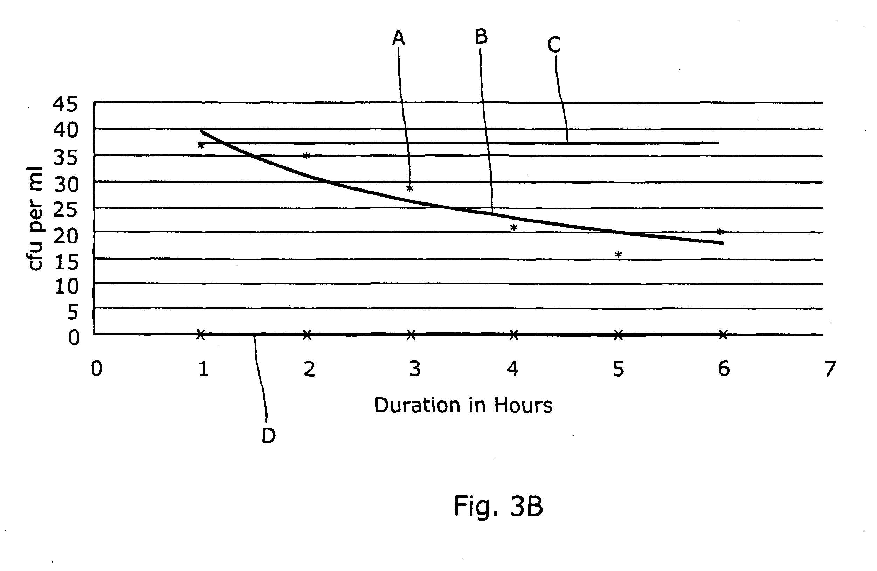

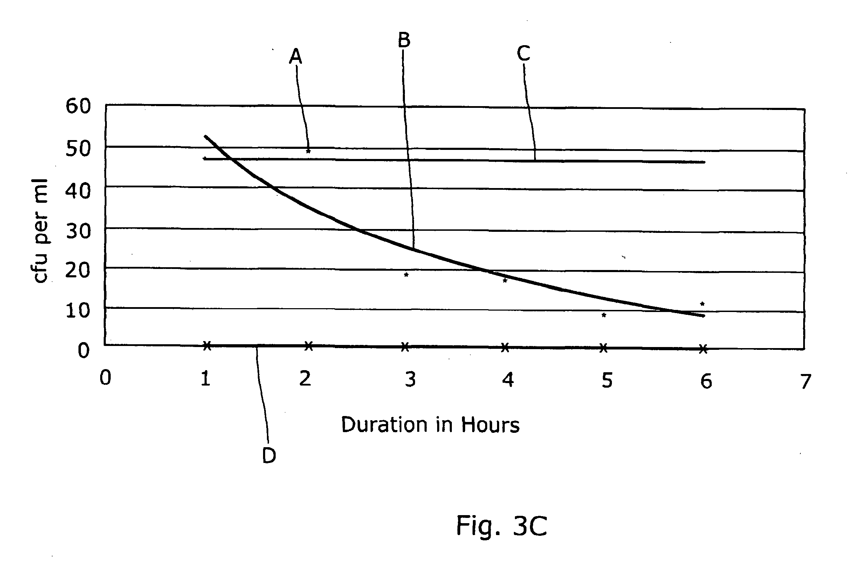

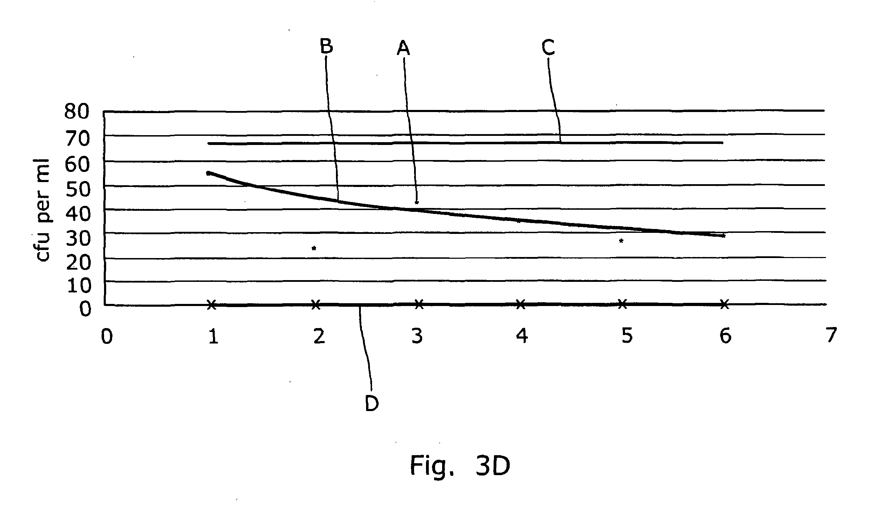

Referring to figure 3, data points are indicated by crosses A, with the logarithmic trend being indicated by line B. The level of the start culture is indicated by line C, while the level of the clean scope (negative control) is indicated by line D. The results show that the microbial populations do not exceed that of packing and gradually reduce to "none detected" over the storage period, that after the designated storage period any residual bacteria (both aerobic and anaerobic microorganisms) decrease and that the mock endoscopes retained their packed integrity for the duration of the test period.

Figures 3B,C and D show corresponding test results for "wet" scopes packed using the method of the invention. As for the dry tests, pieces of 2mm ID PTFE were cut into 1.5m and 2m lengths, sealed in a pack and irradiated to ensure sterility. A piece of tube was then removed from the sterilized pack and aseptically inoculated with a lenticule disc containing approximately 50 cfu of the organism to be used, viz. P.aeruginosa, C.difficile or S.aureus 0.3% Bovine Serum was added to the culture.

Samples were then packed according to the present invention and in a controlled ambient environment at 20°C ± 2°C; however, before packing, bags were inoculated with external rinse water. Inoculated test tubes with each organism type and an one un-inoculated tube(negative control) for each set of shelf life tests were also packed. A positive control set of each organism was also inoculated for each test day, placed in an unsealed bag and left in ambient air conditions. A positive control for each organismon a piece of the test tube [Surrogate Device] was tested for both 1.5m and 2m lengths to establish the amount of bacteria contained in each piece of test tube. All packs were then pressure tested for seal integrity using a pressure/vacuum testing system.

After the set time intervals, and before opening, the seal integrity was again tested using a pressure/vacuum testing system. The test tube pieces [Surrogate Devices], the positive unpacked tubes and the negative packed tubes were then examined after designated storage period for the presence of viable organisms.

Figures 3B-D illustrate that the microbial populations do not exceed that of packing and gradually reduce over the storage period (measured in cfu/ml) for mock endoscopes containing a low level of culture (representative of the maximum allowable AER TVC) of microorganisms packed in the manner set out above and evaluated at regular intervals over a 6 hour period. The test results show that after the designated storage period any residual bacteria (both aerobic and anaerobic microorganisms) steadily decreased and that the mock endoscopes retained their packed integrity for the duration of the test period.

The above packaging method is one step of a total system covering an endoscope from one patient to the next. After removal from a patient, the dirty endoscope is put in a bag and sealed. This helps remove any cross infection risks for both staff and patients when transporting dirty endoscopes from one area to another or from one hospital to another. Bags and trays used for dirty endoscopes may be provided with features, e.g. a red colouring, to distinguish them from bags and trays for use with disinfected devices. Such a red bag gives a clear message to a trained decontamination specialist that the endoscope is not clean and requires reprocessing. Following reprocessing in an AER and possibly a DSC, the clean endoscope is then packaged as set out above.

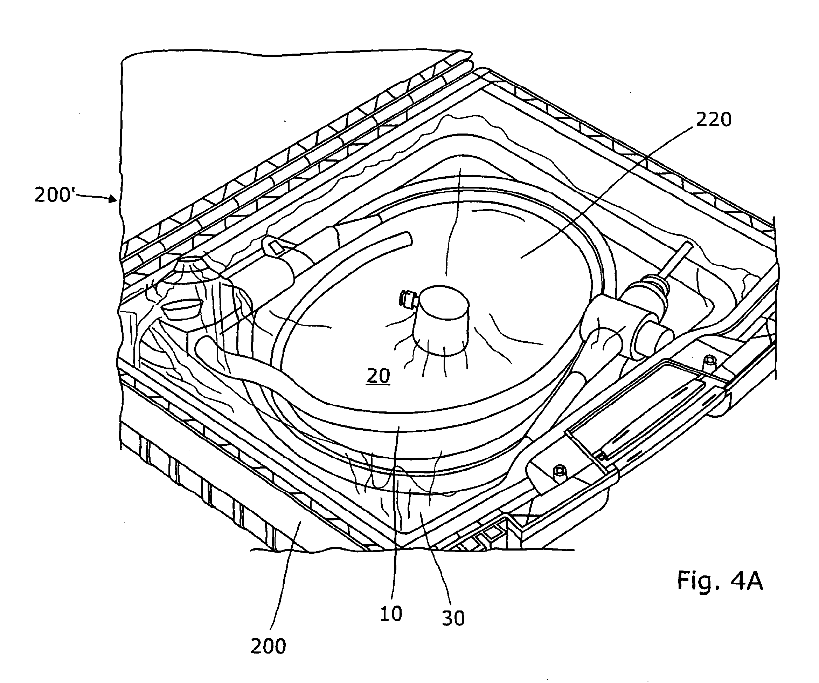

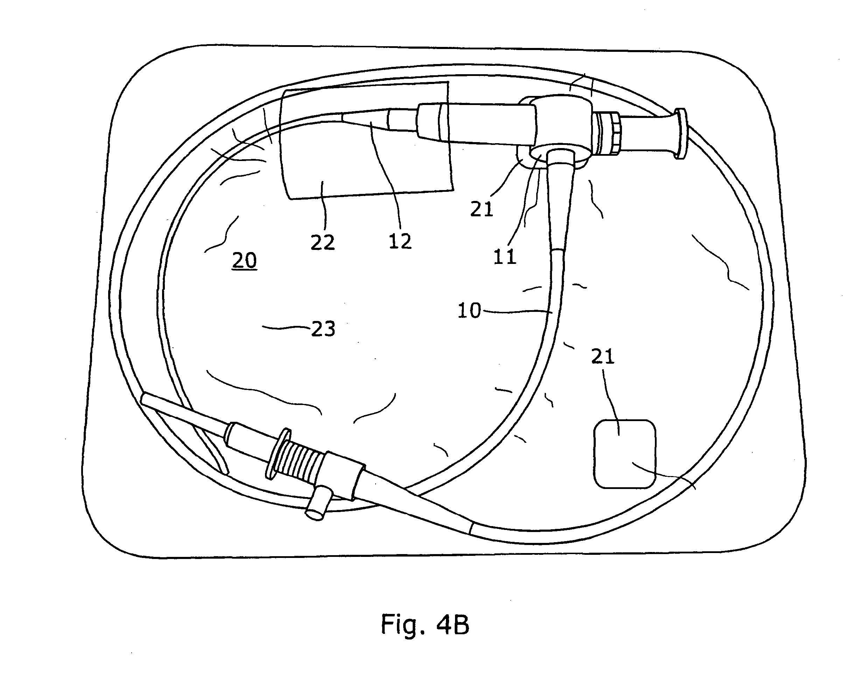

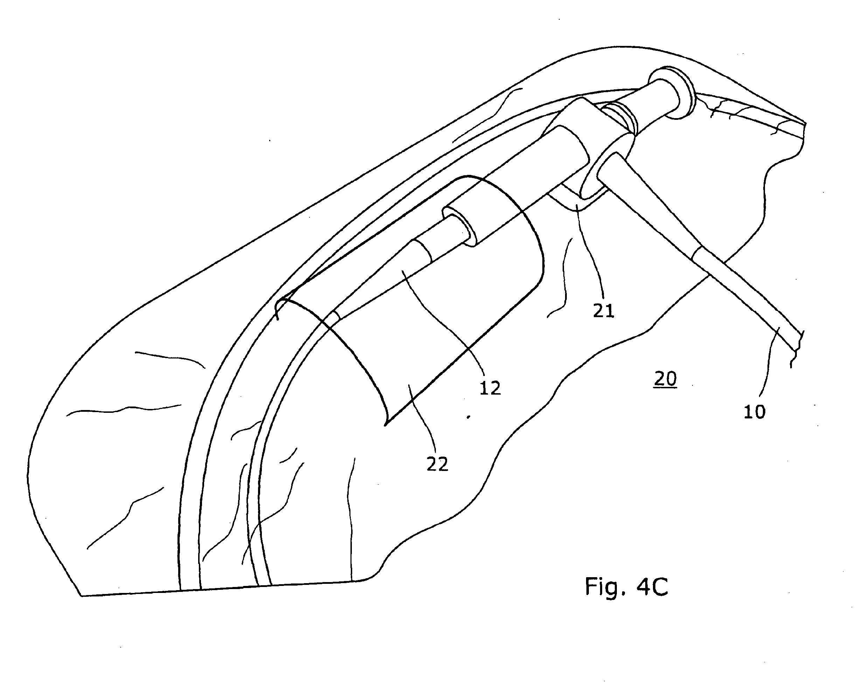

Figure 4A shows the device 10 after packaging in the bag 30, the tray 20 helping to protect the scope from any abnormal contours under the effect of the vacuum. To prevent the bag from tearing against the various protrusions of an endoscope when a vacuum is applied, the bag may be made of heavy gauge rather than thinner, standard gauge material. To avoid undue concentration of force on delicate external surfaces of medical devices such as endoscopes, the supporting surface 23 of the tray 20 may be provided with one or more recesses - indicated at 21 in the plan view of figure 4B - to accommodate protuberant parts 11 of the endoscope 10 and allow the remainder of device to lie substantially flat on the supporting surface of the tray. As also illustrated in the perspective view of figure 4C, the tray 20 may also be provided with one or more bridge pieces 22 to extend over any particularly delicate regions 12 of the device and thereby protect those regions from the force exerted by the material of the bag when evacuated as described above. In the example shown, bridge piece 22 is semicircular in section, having a diameter in the range from about 40mm to about 70mm and a typical length of around 100mm. These bridge pieces may be separate or be formed integrally with a tray. Where the device is an endoscope, a cap may also be provided that permits application of vacuum to the lumens but that prevents application of vacuum to the endoscope sheath. The tray 20 and bridges 22 are made of material, such as polycarbonate, that is able to withstand standard sterilisation procedures, e.g. autoclaving at 134°C for 2 minutes.

In another embodiment, not illustrated, the device may be placed in a further, sterile bag before this is placed in bag 30, thereby ensuring that the scope is not compromised by contact with the tray. However, this inner bag is not sealed in order that the vacuum might be applied to both the inner bag and the bag 30.

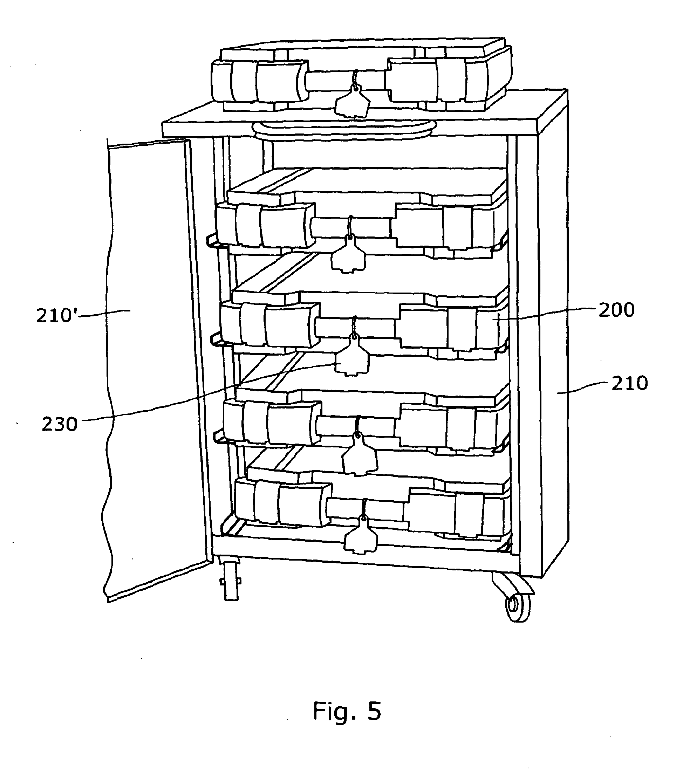

The package is placed in a rigid case 200 having a lid 200', thereby providing further protection of the device and its sealed bag. It will be seen that the use of a tray ensures the package shape fits perfectly into the transportation case every time. As shown in figure 5, yet further protection may be provided by placing the rigid case 200 in an enclosure 210 having a door 210' configured to accommodate a plurality of such cases. Such endoscopes are then ready for use on a patient.

Regulations such as HTM2030 and ISO 15883 may require that any process that produces a final product before use on a patient - a so-called "terminal process" - be fitted with an Independent Monitoring System (IMS) for cycle/process validation and verification. This is a requirement for any AER, DSC or steam sterilizer. Such an IMS system must be independent of the device's (typically microprocessor) controller and to this end is fitted with its own set of probes used to monitor all cycle/process parameters that are critical to the process. Cycle validation data is passed to a PC or mass storage device and information can then be saved to the patient's file, as often required by law.

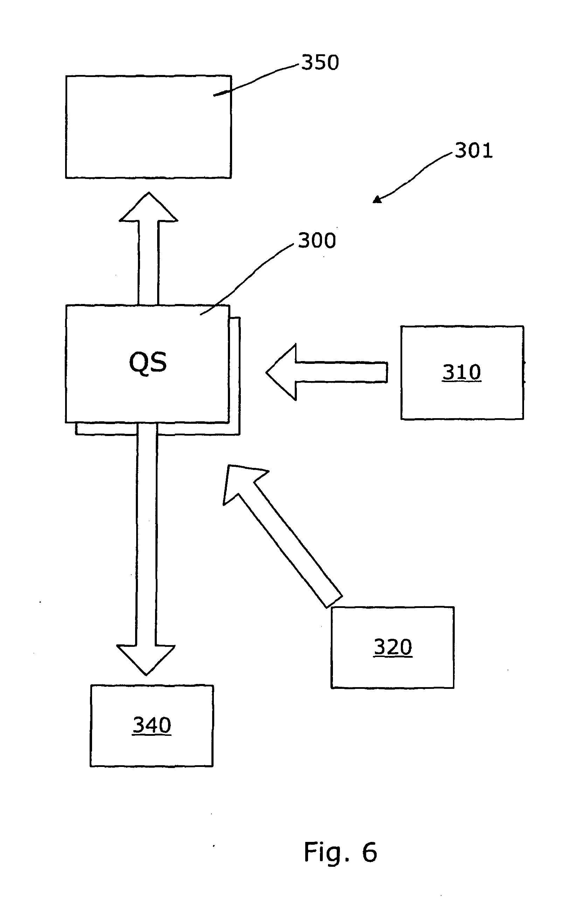

To this end, an independent system 301 of the kind illustrated in figure 6 monitors the vacuum in the bag and the seal heater at the time the bag is hermetically sealed and compares the monitored vacuum with a predetermined critical process value of vacuum. The controller 300 of such an independent monitoring system (IMS) is independent of the packaging device's microprocessor controller and has its own vacuum and seal heater sensors 310 as well as a reader 320 for identifying the packaged endoscope, e.g. by means of a barcode containing the endoscope serial number. The reader may also identify e.g. the operator and the validity period. Depending on the result of said comparison, a signal is generated by the controller that will either raise an alarm or cause one or more validation labels to be printed by printer 340. These labels can be attached to the bag as indicated at 220 in figure 4 and/or to a seal 230 on the case as shown in figure 5. Controller 300 can also pass data to a data storage device 350 for tracking purposes.

The present invention provides a quick and easy method of packaging medical devices such as endoscopes ready for transportation and storage.. This in turn reduces the use of expensive disinfectants used to re-disinfect endoscopes, as well as reducing the number of drying cabinets required. It also reduces the number of times an endoscope is reprocessed in an AER which subjects the endoscope to harsh chemicals and over time takes its toll on the condition of the device.

It should be understood that this invention has been described by way of examples only and that a wide variety of modifications can be made without departing from the scope of the invention.

## Claims

1. Method of packaging a medical device (10), comprising the steps of:
placing the medical device in a bag (30); and
removing air from the bag by applying a vacuum of less than 1 bar;
**characterized by** hermetically sealing the bag (30) while the vacuum is being applied such that the
concentration of oxygen in the hermetically sealed bag is 16% +/- 0.5% by volume.

2. Method of packaging a medical device according to claim 1, the medical device (10) having a sealed chamber (120), comprising the step of applying a vacuum to the interior of the bag (30) such that the pressure difference between the sealed chamber and the interior of the bag does not exceed 1 bar.

3. Method according to any preceding claim and comprising the step of hermetically sealing the bag (30) whilst the vacuum is being applied and such that the vacuum in the bag is about 250mbar below the ambient pressure.

4. Method according to any preceding claim and wherein the step of hermetically sealing the bag (30) comprises the creation of at least two seals (41) in series.

5. Method according to any preceding claim and comprising the step of subjecting the medical device (10) to high level disinfection prior to placing the device in a bag (30).

6. Method according to claim 5 and comprising the step of ensuring that the exposed surfaces of the medical device (10) are dry before placing the medical device in the bag (30).

7. Method according to any preceding claim and comprising the step of placing the medical device (10) on a tray (20) before placing the device and tray into the bag (30).

8. Method according to claim 7 and comprising the step of providing a tray (20) having a supporting surface (23) with a recess (21) formed therein and placing the medical device (10) such that one part of the medical device is supported by the surface and another part of the device lies in the recess.

9. Method according to any preceding claim and comprising the step of monitoring the vacuum in the bag (30) at the time the bag is hermetically sealed, comparing the monitored vacuum with a predetermined value and generating a signal depending on the result of said comparison.

10. Method according to claim 9, wherein the process of monitoring of the vacuum is independent of the process of removing air from the bag (30).

11. Method according to claim 10, wherein the process of removing air from the bag (30) is controlled by a first controller (50,61) and the process of monitoring the vacuum is controlled by a second controller (300), independent of the first controller.

12. Method according to claim 11, wherein the first and second controllers (50,61; 300) are connected to respective independent sensors (61; 310).

13. Method according to any one of claims 9 to 12 and comprising the step of passing data from the second controller (300) to a data storage device (350).

14. Method according to any one of claims 9 to 13 and comprising the step of printing one or more labels corresponding to the signal.

15. Method according to any preceding claim wherein the medical device (10) is an endoscope.

## Patentansprüche

1. Verfahren zum Verpacken einer medizinischen Vorrichtung (10), das die folgenden Schritte beinhaltet:
Platzieren der medizinischen Vorrichtung in einem Beutel (30); und
Entfernen von Luft aus dem Beutel durch Applizieren eines Unterdrucks von weniger als 1 bar;
**gekennzeichnet durch** hermetisches Verschließen des Beutels (30), während der Unterdruck appliziert wird, so dass die Sauerstoffkonzentration in dem hermetisch verschlossenen Beutel 16 +/- 0,5 Vol-% beträgt.

2. Verfahren zum Verpacken einer medizinischen Vorrichtung nach Anspruch 1, wobei die medizinische Vorrichtung (10) eine abgedichtete Kammer (120) aufweist, das den Schritt des Applizierens eines Unterdrucks auf das Innere des Beutels (30) beinhaltet, so dass die Druckdifferenz zwischen der abgedichteten Kammer und dem Innern des Beutels 1 bar nicht übersteigt.

3. Verfahren nach einem vorherigen Anspruch, das den Schritt des hermetischen Verschließens des Beutels (30) beinhaltet, während der Unterdruck darauf appliziert wird, und so, dass der Unterdruck in dem Beutel etwa 250 mbar unter dem Umgebungsdruck liegt.

4. Verfahren nach einem vorherigen Anspruch, wobei der Schritt des hermetischen Verschließens des Beutels (30) das Erzeugen von wenigstens zwei Dichtungen (41) in Serie beinhaltet.

5. Verfahren nach einem vorherigen Anspruch, das den Schritt des Unterziehens der medizinischen Vorrichtung (10) einer hochwirksamen Desinfektion vor dem Platzieren der Vorrichtung in einem Beutel (30) beinhaltet.

6. Verfahren nach Anspruch 5, das den Schritt des Sicherstellens beinhaltet, dass die exponierten Flächen der medizinischen Vorrichtung (10) trocken sind, bevor die medizinische Vorrichtung in dem Beutel (30) platziert wird.

7. Verfahren nach einem vorherigen Anspruch, das den Schritt des Platzierens der medizinischen Vorrichtung (10) auf einer Schale (20) beinhaltet, bevor die Vorrichtung und die Schale in dem Beutel (30) platziert werden.

8. Verfahren nach Anspruch 7, das die Schritte des Bereitstellens einer Schale (20) mit einer Auflagefläche (23) mit einer darin ausgebildeten Aussparung (21) und des Platzierens der medizinischen Vorrichtung (10) auf eine solche Weise beinhaltet, dass ein Teil der medizinischen Vorrichtung auf der Fläche gelagert ist und ein anderer Teil der Vorrichtung in der Aussparung liegt.

9. Verfahren nach einem vorherigen Anspruch, das die Schritte des Überwachens des Unterdrucks in dem Beutel (30) zum Zeitpunkt des hermetischen Verschließens des Beutels, des Vergleichens des überwachten Unterdrucks mit einem vorbestimmten Wert und des Erzeugens eines Signals je nach dem Ergebnis des genannten Vergleichs beinhaltet.

10. Verfahren nach Anspruch 9, wobei der Vorgang des Überwachens des Unterdrucks von dem Vorgang des Entfernens von Luft aus dem Beutel (30) unabhängig ist.

11. Verfahren nach Anspruch 10, wobei der Vorgang des Entfernens von Luft aus dem Beutel (30) von einer ersten Steuerung (50, 61) gesteuert wird und der Vorgang des Überwachens des Unterdrucks von einer zweiten Steuerung (300) unabhängig von der ersten Steuerung gesteuert wird.

12. Verfahren nach Anspruch 11, wobei die erste und zweite Steuerung (50, 61; 300) mit jeweiligen unabhängigen Sensoren (61; 310) verbunden sind.

13. Verfahren nach einem der Ansprüche 9 bis 12, das den Schritt des Leitens von Daten von der zweiten Steuerung (300) zu einer Datenspeichervorrichtung (350) beinhaltet.

14. Verfahren nach einem der Ansprüche 9 bis 13, das den Schritt des Druckens von einem oder mehreren Schildern beinhaltet, die dem Signal entsprechen.

15. Verfahren nach einem vorherigen Anspruch, wobei die medizinische Vorrichtung (10) ein Endoskop ist.

## Revendications

1. Procédé d'emballage d'un dispositif médical (10), comprenant les étapes consistant à :
placer le dispositif médical dans un sachet (30) ; et
évacuer l'air hors du sachet en appliquant une dépression qui est inférieure à 1 bar,
**caractérisé par** un scellement hermétique du sachet (30) pendant que la dépression est appliquée, de telle sorte que la concentration en oxygène dans le sachet hermétiquement scellé soit de 16 % +/-0,5 % en volume.

2. Procédé d'emballage d'un dispositif médical selon la revendication 1, le dispositif médical (10) possédant une chambre scellée (120), comprenant l'étape consistant à appliquer une dépression au volume interne du sachet (30) de telle sorte que la différence de pression entre la chambre scellée et le volume interne du sachet ne soit pas supérieure à 1 bar.

3. Procédé selon l'une quelconque des revendications précédentes, et comprenant l'étape consistant à sceller hermétiquement le sachet (30) pendant que la dépression est appliquée et de telle sorte que la dépression dans le sachet soit inférieure d'environ 250 mbars par rapport à la pression ambiante.

4. Procédé selon l'une quelconque des revendications précédentes, et l'étape consistant à sceller hermétiquement le sachet (30) comprenant la création d'au moins deux joints d'étanchéité (41) en série.

5. Procédé selon l'une quelconque des revendications précédentes, et comprenant l'étape consistant à soumettre le dispositif médical (10) à une désinfection de niveau élevé avant de placer le dispositif dans un sachet (30).

6. Procédé selon la revendication 5, et comprenant l'étape consistant à s'assurer que les surfaces exposées du dispositif médical (10) sont sèches avant de placer le dispositif médical dans le sachet (30).

7. Procédé selon l'une quelconque des revendications précédentes, et comprenant l'étape consistant à placer le dispositif médical (10) sur un plateau (20) avant de placer le dispositif et le plateau dans le sachet (30).

8. Procédé selon la revendication 7, et comprenant les étapes consistant à mettre à disposition un plateau (20) possédant une surface de support (23) avec un évidement (21) qui est formé dans celle-ci, et à placer le dispositif médical (10) de telle sorte qu'une partie du dispositif médical soit soutenue par la surface et qu'une autre partie du dispositif se trouve dans l'évidement.

9. Procédé selon l'une quelconque des revendications précédentes, et comprenant les étapes consistant à surveiller la dépression dans le sachet (30) au moment où le sachet est hermétiquement scellé, à comparer la dépression surveillée à une valeur prédéterminée, et à générer un signal en fonction du résultat de ladite comparaison.

10. Procédé selon la revendication 9, le processus de surveillance de la dépression étant indépendant du processus d'évacuation de l'air hors du sachet (30).

11. Procédé selon la revendication 10, le processus d'évacuation de l'air hors du sachet (30) étant piloté par un premier contrôleur (50, 61) et le processus de surveillance de la dépression étant piloté par un deuxième contrôleur (300) lequel est indépendant du premier contrôleur.

12. Procédé selon la revendication 11, les premier et deuxième contrôleurs (50, 61 ; 300) étant connectés à des capteurs respectifs indépendants (61 ; 310).

13. Procédé selon l'une quelconque des revendications 9 à 12, et comprenant l'étape consistant à faire passer des données à partir du deuxième contrôleur (300) vers un dispositif de stockage de données (350).

14. Procédé selon l'une quelconque des revendications 9 à 13, et comprenant l'étape consistant à imprimer une ou plusieurs étiquettes correspondant au signal.

15. Procédé selon l'une quelconque des revendications précédentes, le dispositif médical (10) étant un endoscope.
